(19) Europäisches Patentamt
European Patent Office
Office européen des brevets

(11) EP 3 603 613 A1

(12) EUROPEAN PATENT APPLICATION
published in accordance with Art. 153(4) EPC

(43) Date of publication:
**05.02.2020 Bulletin 2020/06**

(21) Application number: **19780136.8**

(22) Date of filing: **10.05.2019**

(51) Int Cl.:
***A61K 8/49*** *(2006.01)*
***A23L 33/10*** *(2016.01)*
***A23K 20/121*** *(2016.01)*
***A61K 31/352*** *(2006.01)*
***A61Q 19/08*** *(2006.01)*
***A61P 17/00*** *(2006.01)*

(86) International application number:
**PCT/KR2019/005652**

(87) International publication number:
**WO 2019/225891 (28.11.2019 Gazette 2019/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.05.2018 KR 20180058915**

(71) Applicant: **Phyto Corporation Inc.**
**Seoul 08826 (KR)**

(72) Inventors:
- **KIM, Deuk Hoi**
  **Goyang-si, Gyeonggi-do 10551 (KR)**
- **KWEON, Mee Hyang**
  **Seoul 07000 (KR)**
- **PARK, Seon Yeong**
  **Seoul 02097 (KR)**
- **LEE, Sang Mee**
  **Seoul 03392 (KR)**

(74) Representative: **J A Kemp LLP**
**14 South Square**
**Gray's Inn**
**London WC1R 5JJ (GB)**

# (54) SKIN ANTI-AGING COMPOSITION CONTAINING IRILIN B

(57) The present invention relates to a skin anti-aging composition containing Irilin B (5,7,2'-trihydroxy-6-methoxyisoflavone) or a salt thereof, wherein Irilin B isolated from *Salicornia* spp. may be used itself or in the form of a salt thereof, and shows effects of enhancing skin elasticity, preventing and reducing wrinkles, or maintaining skin moisturization. The skin anti-aging composition of the present invention can be prepared in various forms, such as a cosmetic composition, a food composition, a feed composition, or a pharmaceutical composition.

Fig 1

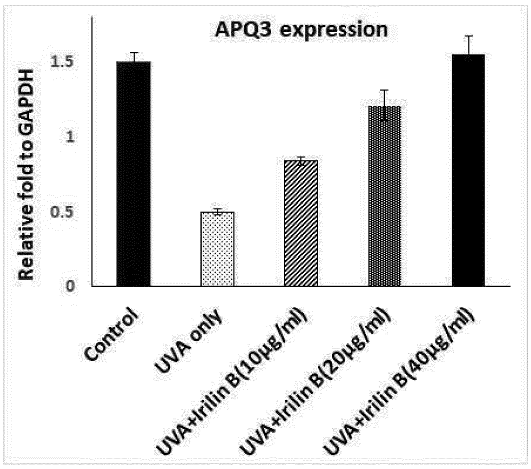


EP 3 603 613 A1

SEQUENCE LISTING

<110> PHYTO CORPORATION INC.

<120> SKIN ANTI-AGING COMPOSITION CONTAINING IRILIN B

<130> N417908EP

<150> KR 10-2018-0058915
<151> 2019-05-10

<160> 7

<170> PatentIn version 3.5

<210> 1
<211> 20
<212> DNA
<213> Artificial Sequence

<220>
<223> AQP3-F

<400> 1
agacagcccc ttcaggattt 20

<210> 2
<211> 20
<212> DNA
<213> Artificial Sequence

<220>
<223> AQP3-R

<400> 2
tcccttgccc tgaatactgg 20

<210> 3
<211> 20
<212> DNA
<213> Artificial Sequence

<220>
<223> HAS2-F

<400> 3
taaggtgttg tgtgtgactg 20

<210> 4
<211> 21
<212> DNA
<213> Artificial Sequence

<220>
<223> HAS2-R

<400> 4
cagaatccaa acagacagtt c 21

```
<210>  5
<211>  20
<212>  DNA
<213>  Artificial Sequence

<220>
<223>  GAPDH-F

<400>  5
accacagtcc atgccatcac                                                 20


<210>  6
<211>  20
<212>  DNA
<213>  Artificial Sequence

<220>
<223>  GAPDH-R

<400>  6
tccaccaccc tgtttcttta                                                 20


<210>  7
<211>  4
<212>  PRT
<213>  Artificial Sequence

<220>
<223>  Synthetic oligopeptide


<220>
<221>  MOD_RES
<222>  (1)..(1)
<223>  Amino terminal methoxysuccinyl

<220>
<221>  MOD_RES
<222>  (4)..(4)
<223>  Carboxy terminal p-nitroanilide

<400>  7

Ala Ala Pro Val
1
```

## Description

### Technical Field

The present invention relates to a skin anti-aging composition containing Irilin B. More specifically, the present invention relates to a skin anti-aging composition containing Irilin B as an active ingredient and exhibiting functions of skin wrinkle reduction, skin wrinkle prevention, skin elasticity enhancement or skin moisturization.

### Background Art

Collagen is a major matrix protein produced in fibroblasts of the skin and exists in the extracellular matrix. The important functions of collagen are known to be providing mechanical rigidity of the skin, resistance of connective tissues and binding strength of tissues, support of cell adhesion, cell division and differentiation (upon organism growth or wound healing). Such collagen is reduced by age and photo-aging resulting from ultraviolet irradiation, and the reduction of collagen is promoted by an increase in activity of collagenase that degrades collagen. The reduction of collagen is known to be highly associated with the formation of skin wrinkles.

In addition, elastic fibers form crosslinks together with collagen, contribute to skin elasticity, and correspond to an important skin component in wrinkle formation. The deficiency and agglomeration of elastic fibers and a marked increase in activity of the elastin degradation enzyme elastase have been revealed to be factors to form skin wrinkles and decrease elasticity. Elastase is a unique enzyme that can degrade elastin. It has been known that the inhibition of elastase can fundamentally reduce skin wrinkles and mitigate elasticity decrease.

Meanwhile, collagen and elastic fibers are matrix proteins that play an important role in moisture retention in the dermis layer. These matrix proteins suck moisture and increase the moisture retention capacity inside a structure formed thereby, thereby keeping the skin properly moisturized. Resultantly, collagen and elastin are involved in maintaining moisturizing functions to keep skin elasticity and retain skin moisture. Therefore, the inhibition of collagenase activity and elastase activity may be an important measure to reduce skin wrinkles, enhance skin elasticity, and maintain skin moisturization.

Meanwhile, skin moisturization may be achieved according to various mechanisms and the accompanying material functions. The materials showing moisturizing functions in cosmetic products may be largely classified, according to the action mechanism, into materials that supply moisture by retaining moisture, materials that inhibit moisture evaporation or loss from the skin, materials that stimulate the formation of moisturizing factors in the skin, and the like.

In recent years, materials that show moisturizing functions by enhancing intercellular spacing or adhesion have also been developed. The intracellular adhesion has very important functions, such as intercellular junction, blocking release of moisture from the skin, blocking inflow of foreign substances, and blocking extracellular fluids through the dermis layer. Especially, the expression levels of factors aquaporin 3 (AQP3) and hyaluronic acid synthase 2 (HAS2), which are skin cell moisture regulating factors and moisturizing factors involved in cell moisture migration, are known to be important biomarkers to identify skin moisturizing functions. Therefore, the compounds that increase the expression levels of AQP3 and HAS2 can be used as anti-aging materials that maintain skin moisturization.

It has recently been argued that the differentiation of the stratum corneum is important to prevent drying of skin moisture. It has been revealed that the skin moisture retaining barrier can be retained only when keratinocytes of the stratum basale normally differentiate into corneocytes of the outermost stratum corneum. That is, during keratinization, the cells produce natural moisturizing factors and intercellular lipids, with the result that the stratum corneum becomes rigid and flexible and thus can function as a barrier.

The tendency of skin to dry with people aging may be physiologically construed as a longer time to exfoliate the stratum corneum, the deterioration in the lipid synthesis ability of epidermal cells, and reductions in moisturizing factors and lipid levels in the stratum corneum. Therefore, appropriate approaches are to induce skin barrier enhancement by promoting the differentiation of keratinocytes and to reduce skin wrinkles and suppress aging through skin moisturization by promoting skin moisture retaining functions and skin protecting functions.

There were various attempts to search for natural substances having a skin wrinkle reducing effect by inhibiting decreases in collagen and elastin. To use wrinkle reducing effects of collagen and elastin, products obtained by combining collagen and elastin with a composition for external application to skin, such as a cosmetic product or ointment, have been released. However, these products are used by applying the protein macromolecules collagen and elastin *per se* to the skin surface, and thus percutaneous absorption of collagen and elastin is difficult, resulting in no substantial wrinkle reducing effects.

To solve these problems, there has been a growing interest in collagenesis-stimulating substances. Current collagenesis-stimulating substances are vitamin C, retinol (retinoid), transforming growth factor (TGF), an animal placenta-derived protein (JP 8-231370), betulinic acid (JP8-208424), chlorella extracts (JP9-40523, JP10-36283, stimulating fibroblast proliferation), and adenosine or Kojic acid derived from mold and yeast that inhibit enzymatic degradation of

such molecules.

However, such substances are limited in the amount of use due to safety problems, such as irritation and redness upon application to the skin, or a wrinkle reducing effect can be substantially expected due to slight effects of the substances. Therefore, there is an urgent need for the development of novel wrinkle-reducing components, which are safe for living bodies and have excellent wrinkle reduction and moisturizing effects compared with conventional anti-wrinkle compositions.

**Detailed Description of the Invention**

**Technical Problem**

The present inventors endeavored to develop an anti-aging composition for skin wrinkle reduction and skin moisture retention. As a result, the present inventors have developed a skin anti-aging composition containing Irilin B or a salt thereof.

Irilin B inhibited activity of collagenase and elastase, which are enzyme to promote skin wrinkle formation and elasticity deterioration. Furthermore, Irilin B restored the expression of aquaporin 3 (AQP3) and hyaluronic acid synthase 2 (HAS2), skin moisturizing factors.

Therefore, the present inventors verified that Irilin B are remarkably excellent in skin anti-aging, and thus completed the present invention.

An aspect of the present invention is to provide a skin anti-aging composition containing Irilin B or a salt thereof.

Another aspect of the present invention is to provide a method for skin anti-aging using Irilin B.

Still another aspect of the present invention relates to the use of Irilin B or a salt thereof for skin anti-aging.

**Technical Solution**

The present inventors endeavored to develop an anti-aging composition for skin wrinkle reduction and skin moisture retention, and as a result, the present inventors have developed a skin anti-aging composition containing Irilin B or a salt thereof.

The present inventors assumed that Irilin B can inhibit the activity of collagenase and elastase, which are enzymes to promote skin wrinkle formation and elasticity deterioration.

To investigate the assumption, the anti-collagenase activity and anti-elastase activity of Irilin B compared with known positive control compounds were measured. As validated in the following example, Irilin B inhibited the enzyme activity of collagenase and elastase, and thus Irilin B was proved to have effects of reducing skin wrinkles, preventing skin wrinkles, and enhancing skin elasticity.

Meanwhile, a decrease in moisturization in the skin is an important aging factor during skin aging. The expression levels of the skin moisturizing factors, aquaporin 3 (AQP3) and hyaluronic acid synthase 2 (HAS2), were compared by measurement using skin keratinocytes. As validated in the following examples, Irilin B restored the UVA-reduced expression of AQP3 and HAS2 to normal levels.

In addition, the differentiation promoting effect of Irilin B on the keratinocytes that play an important role in skin moisturization and aging prevention was investigated. As a result, Irilin B has been provide to maintain skin moisturization, enhancing skin moisturization, and reduce skin wrinkles.

Hereinafter, the present invention will be described in detail.

An aspect of the present invention relates to a skin anti-aging composition containing Irilin B (5,7,2'-trihydroxy-6-methoxyisoflavone) represented by chemical formula 1 below or a salt thereof.

[Chemical Formula 1]

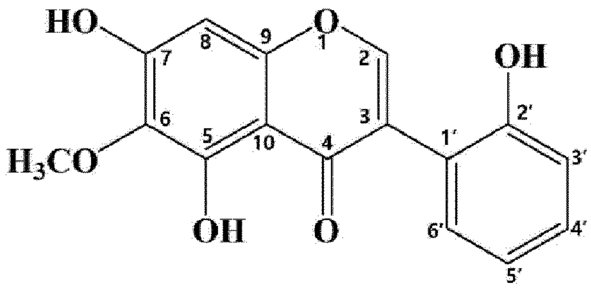

The chemical formula of Irilin B (5,7,2'-trihydroxy-6-methoxyisoflavone) is $C_{16}H_{12}O_6$, with a molecular weight of about 300. Irilin B is an isoflavonoid-based compound having a methoxyl group.

Irilin B was first discovered and named as a plant stress metabolite compound in the family Iridaceae in 1991. Irilin B was isolated as an anti-thrombotic and whitening compound from *Salicornia* spp., which are halophytes, by the present inventors in 2016.

Irilin B can be isolated as a light yellow powder from plants, such as *Salicornia* spp., *Iris ensata*, and *Iris sanguinea*, or obtained from organic synthesis. Preferably, Irilin B may be represented by a compound of chemical formula 1 (5,7,2'-trihydroxy-6-methoxyisoflavone) below:

[Chemical Formula 1]

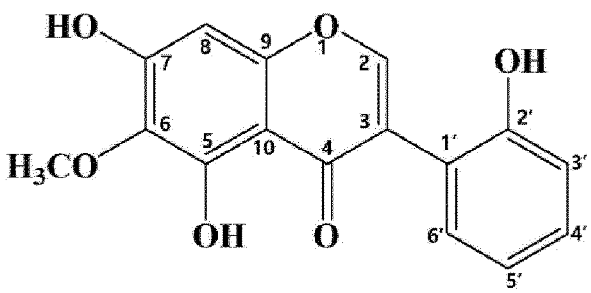

An embodiment of the present invention relates to a skin anti-aging composition containing Irilin B as an active ingredient for skin elasticity enhancement, skin wrinkle prevention, skin wrinkle reduction, and/or skin moisturization.

As used herein, the term "skin anti-aging" is used as a meaning including skin wrinkle reduction, skin wrinkle prevention, skin elasticity enhancement, or skin moisturization.

As used herein, the term "containing as an active ingredient" refers to containing an amount sufficient to attain efficacy or activity of Irilin B or a salt thereof.

The Irilin B contained in the composition of the present invention may be a compound isolated from *Salicornia* spp., natural plant materials, but is not limited thereto, or may be a synthetic compound.

The upper limit of the amount of Irilin B or a salt thereof contained in the composition of the present invention may be selected within an appropriate range by a person skilled in the art.

Irilin B used as an active ingredient in the present may be used itself or in the form of a salt thereof, for example, a pharmaceutically acceptable salt thereof, but is not limited thereto.

The salt is preferably an acid addition salt formed by a free acid.

The free acid may include an inorganic acid and an organic acid.

The organic acid includes, but is not limited to, citric acid, acetic acid, lactic acid, tartaric acid, maleic acid, fumaric acid, formic acid, propionic acid, oxalic acid, tripleuroacetic acid, benzoic acid, gluconic acid, methanesulfonic acid, glycolic acid, succinic acid, 4-toluenesulfonic acid, glutamic acid, and aspartic acid.

In addition, the inorganic acid includes hydrochloric acid, bromic acid, sulfuric acid, and phosphoric acid, but is not limited thereto.

*Salicornia* spp. have long been used for an edible purpose and as a folk drug, and thus Irilin B of the present invention isolated therefrom can be expected to have no toxicity and side effects.

Therefore, Irilin B or a salt thereof cannot only be used as pharmaceutical, food, and cosmetic compositions, but be also developed as animal medicines and functional feeds.

According to an embodiment of the present invention, the skin anti-aging composition of the present invention is a cosmetic composition.

The cosmetic composition of the present invention may be formulated in any dosage form that is ordinarily prepared in the art, and examples thereof may include a solution, a suspension, an emulsion, a paste, a gel, a cream, a lotion, a powder, a soap, a surfactant-containing cleansing, an oil, a powder foundation, an emulsion foundation, a wax foundation, and a spray, but are not limited thereto.

More specifically, the cosmetic composition of the present invention may be prepared in a dosage form of an emollient lotion, a nourishing lotion, a nourishing cream, a massage cream, an essence, an eye cream, a cleansing cream, a cleansing foam, a cleansing water, a pack, a spray, or a powder.

In cases where the dosage form of the present invention is a paste, a cream, or a gel, animal oils, vegetable oils, wax, paraffin, starch, tragacanth, cellulose derivatives, polyethylene glycol, silicone, bentonite, silica, talc, or zinc oxide may be used as a carrier ingredient.

In cases where the dosage form of the present invention is a power or a spray, lactose, talc, silica, aluminum hydroxide, calcium silicate, or a polyamide powder may be used as a carrier ingredient. Especially, in cases where the dosage form of the present invention is a spray, such a spray may further contain a propellant, such as chlorofluorohydrocarbon, propane/butane, or dimethyl ether.

In cases where the dosage form of the cosmetic composition is a solution or an emulsion, a solvent, a solubilizer, or an emulsifier may be used as a carrier ingredient, and examples thereof include water, ethanol, isopropanol, ethyl carbonate, ethyl acetate, benzyl alcohol, benzyl benzoate, propylene glycol, 1,3-butyl glycol oil, glycerol aliphatic esters, polyethylene glycol, or fatty acid esters of sorbitan.

In cases where the dosage form of the present invention is a suspension, a liquid diluent (such as water, ethanol, or propylene glycol), a suspending agent (such as ethoxylated isostearyl alcohol, polyoxyethylene sorbitol ester, or polyoxyethylene sorbitan ester), microcrystalline cellulose, aluminum metahydroxide, bentonite, agar, or tragacanth, may be used as a carrier ingredient.

In cases where the dosage form of the present invention is a surfactant-containing cleansing, an aliphatic alcohol sulfate, an aliphatic alcohol ether sulfate, sulfosuccinate monoester, isethionate, an imidazolium derivative, methyl taurate, sarcosinate, a fatty acid amide ether sulfate, an alkyl amido betaine, an aliphatic alcohol, fatty acid glyceride, fatty acid diethanolamide, vegetable oil, a lanoline derivative, or ethoxylated glycerol fatty acid ester may be used as a carrier ingredient.

The ingredients contained in the cosmetic composition of the present invention include, in addition to the active ingredient and the carrier ingredient, ingredients that are usually used in the cosmetic composition, and for example, may include ordinary adjuvants, such as an anti-oxidant, a stabilizer, a solubilizer, a vitamin, a pigment, and a flavoring agent.

The amount of Irilin B or a salt thereof as an active ingredient in the cosmetic composition of the present invention is not particularly limited, and preferably, the Irilin B or a salt thereof is contained in an amount sufficient to attain efficacies of reducing skin wrinkles, enhancing skin elasticity and/or maintaining skin moisturization.

According to still another embodiment of the present invention, the skin anti-aging composition of the present invention is a food or feed composition.

The composition of the present invention, when being a food or feed composition, may be prepared in the form of a powder, granules, a tablet, a capsule, or a beverage, for example, candy, drink, gum, or tea, but is not limited thereto.

The composition of the present invention, when being a food composition, may be in the form of a vitamin composite or a health supplement food, but is not limited thereto.

The food or feed composition of the present invention may contain not only Irilin B as an active ingredient but also the ingredients that are usually added in the manufacture of a food or feed, and may contain, for example, a protein, a carbohydrate, a fat, a nutrient, a seasoning, and a flavoring agent, but is not limited thereto.

The foregoing carbohydrate may include monosaccharides (e.g., glucose, fructose, etc.); disaccharides (e.g., maltose, sucrose, oligosaccharides, etc.); and polysaccharides (e.g., ordinary sugars (such as dextrin and cyclodextrin), sugar alcohols (such as xylitol, sorbitol, and erythritol)), and natural flavoring agents (thaumatin and a stevia extract (e.g., Rebaudioside A, glycyrrhizin, etc.)) and synthetic flavoring agents (e.g., saccharin, aspartame, etc.) may be used as flavoring agents, but are not limited thereto.

For example, the food composition of the present invention, when manufactured as a drink, may further contain citric acid, liquefied fructose, sugar, glucose, acetic acid, malic acid, fruit juice, a *Eucommia ulmoides* extract, a jujube extract, and/or a licorice extract.

According to still another embodiment of the present invention, the skin anti-aging composition of the present invention is a pharmaceutical composition.

The pharmaceutical composition of the present invention may contain a pharmaceutically acceptable carrier.

The pharmaceutically acceptable carrier is ordinarily used in the formulation, and examples thereof may include, but are not limited to, lactose, dextrose, sucrose, sorbitol, mannitol, starch, acacia gum, calcium phosphate, alginate,

gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and/or mineral oil.

The pharmaceutical composition of the present invention may further contain, in addition to the above ingredients, a lubricant, a wetting agent, a sweetening agent, a flavoring agent, an emulsifier, a suspending agent, and/or a preservative, and the like, but is not limited thereto.

Suitable pharmaceutically acceptable carriers and preparations are described in detail in Remington's Pharmaceutical Sciences (19th ed., 1995).

The pharmaceutical composition of the present invention may be administered orally or parenterally, and examples of parenteral administration may include intravenous injection, subcutaneous injection, intramuscular injection, intraperitoneal injection, and percutaneous administration.

The appropriate dose of the pharmaceutical composition of the present invention varies depending on factors, such as a formulation method, a manner of administration, patient's age, body weight, gender, and morbidity, food, a time of administration, a route of administration, an excretion rate, and response sensitivity. An ordinarily skilled practitioner can easily determine and prescribe an effective dose for desired treatment or prevention.

According to a preferable embodiment of the present invention, the daily dose of the pharmaceutical composition of the present invention is 0.001-10000 **mg/kg.**

The pharmaceutical composition of the present invention may be formulated using a pharmaceutically acceptable carrier and/or excipient by a method that may be easily performed by a person having ordinary skill in the art to which the invention pertains, so that the composition may be prepared in a unit dosage form or may be contained in a multi-dose container.

The dosage form may be a solution, suspension, or an emulsion in an oily or aqueous medium, or an extract, a powder, granules, a tablet, or a capsule, and may further contain a dispersing agent or a stabilizer.

The skin anti-aging composition of the present invention may contain 0.00001-30 wt% of Irilin B or a salt thereof relative to the entire composition.

Another aspect of the present invention relates to a composition containing Irilin B or a salt thereof for external application to skin.

Still another aspect of the present invention relates to a method for suppressing skin aging or improving skin condition, the method including administering to a subject a skin anti-aging composition containing Irilin B (5,7,2'-trihydroxy-6-methoxyisoflavone) or a salt thereof.

As used herein, the term "administration" refers to providing a predetermined substance to a subject in an appropriate manner. As for the administration route of the skin anti-aging composition of the present invention, the composition may be orally or parenterally administered through all the general routes as long as the composition can reach a target tissue. In addition, the skin anti-aging composition of the present invention may be administered using any apparatus that can deliver an active ingredient to target cells.

As used herein, the term "subject" refers to, for example, but is not particularly limited to, a human, monkey, cow, horse, sheep, pig, chicken, turkey, quail, cat, dog, mouse, rat, rabbit, or guinea pig, preferably a mammal, and more preferably a human.

Still another aspect of the present invention relates to the use of Irilin B or a salt thereof for skin anti-aging.

## Advantageous Effects

The present invention relates to a skin anti-aging composition containing Irilin B (5,7,2'-trihydroxy-6-methoxyisoflavone) or a salt thereof. Irilin B may be used itself or in the form of a salt thereof, and shows effects of enhancing skin elasticity, preventing and reducing wrinkles, or maintaining skin moisturization. The skin anti-aging composition of the present invention can be prepared in various forms, such as a cosmetic composition, a food composition, a feed composition, or a pharmaceutical composition.

## Brief Description of the Drawings

FIG. 1 is a graph showing the reduction of AQP3 expression by UAV irradiation and restoring effects by Irilin B according to an embodiment of the present invention.

FIG. 2 is a graph showing the reduction of HAS2 expression by UAV irradiation and restoring effects by Irilin B according to an embodiment of the present invention.

## Mode for Carrying Out the Invention

Hereinafter, the present invention will be described in more detail with reference to the following examples. However, these examples are given for illustrating the present invention, and the scope of the present invention is not limited thereto.

### Example 1: Irilin B

The Irilin B compound used in the present test was purely isolated from an enzymatic hydrolysis product and an ethanol extract of desalted *Salicornia spp.* (Korean Patent Registration No. 10-1812319, molecular weight: 300, chemical formula: $C_{16}H_{12}O_6$, morphology: light yellow powder). Retinol, a collagenase inhibitor, and quercetin, an elastase inhibitor, which are used as positive controls, and transforming growth factor-β (TGF-β), a cytokinin for cell differentiation, were purchased from Sigma Co.

### Example 2: Measurement of anti-collagenase activity

Collagenase, used in the measurement of enzyme activity, was isolated from mouse pancreases. The synthetic peptide, N-(3-[2-Furyl]acryloyl)-Leu-Gly-Pro-Ala, was used as a substrate compound for an enzymatic reaction. A solution containing 50 mM Tricine, 10 mM $CaCl_2 \cdot H_2O$, and 400 mM NaCl (pH 7.5) dissolved therein was used as a buffer solution. The synthetic substrate was dissolved to a concentration of 10 nM in the buffer solution, and then finally diluted to 75 μM. Collagenase was dissolved to a concentration of 1 unit/ml in tertiary distilled water at 4°C. Upon the test, the collagenase diluted to a final concentration of 0.05 unit/ml in the buffer solution was used. Irilin B used to evaluate the anti-collagenase activity thereof had a concentration of 5, 10, or 20 μg/ml. In addition, the concentration of retinol (Sigma Co.), which was a positive control known as an anti-collagenase substance, was 10 μg/ml or 20 μg/ml in the test. The reaction was carried out in 96-well UV plates (Corning Co.) at room temperature for 10 minutes. The absorbance was measured at 345 nm at intervals of 1 minute using a microplate spectrophotometer (Bio-Rad). The enzyme activity was determined by obtaining the maximal slope of absorbance over time. All enzymatic reactions were repeatedly carried out three times, and expressed as average values. The anti-collagenase effects of Irilin B are shown in Table 1. The anti-collagenase activity was calculated as below:

[Calculation Formula]

Anti-collagenase activity (%) = [(absolute value of slope in control - absolute value of slope in test sample) / absolute value of slope in control] X 100

[Table 1]

| Sample name | Concentration (μg/ml) | Enzyme activity (Absolute value of maximal slope of absorbance over time at 345 nm) | Anti-collagenase activity (%) |
|---|---|---|---|
| Irilin B | 5 | 0.54 | 33.33±1.25 |
| | 10 | 0.41 | 49.38±2.87 |
| | 20 | 0.30 | 62.96±3.12 |
| Positive control (Retinol) | 10 | 0.67 | 17.28±0.85 |
| | 20 | 0.56 | 30.86±2.03 |
| Control (DMSO) | 20 | 0.81 | - |

As can be seen from the results of Table 1 above, Irilin B showed anti-collagenase activity (33.33%, 49.38%,

and 62.96%) in a dose dependent manner (5, 10, and 20 μg/ml). It was confirmed that Irilin B showed significantly excellent anti-collagenase activity (by about 2.05-fold) compared with retinol (30.86%, 20 μg/ml), a positive control, tested at the same concentration in the same conditions. That is, it was confirmed that Irilin B had very excellent anti-collagenase activity, and thus can be used for skin anti-aging cosmetics for skin wrinkle reduction and skin elasticity and regeneration.

**Example 3: Measurement of anti-elastase activity**

The anti-elastase activity (elastase being an elastin degradation enzyme) was tested as below. The elastase isolated from human leukocytes were used. The synthetic oligopeptide, MeOSuc-Ala-Ala-Pro-Val-pNA, was used as a substrate of an enzyme reaction. A 100 mM Tris (pH 7.5) solution was used as a buffer solution. Elastase was finally used at 0.2 mU using the buffer solution. In addition, the synthetic substrate of elastase was made into a 100 mM solution using DMSA, and then diluted to a final concentration of 0.5 mM in the buffer solution. Irilin B used to evaluate the anti-elastase activity had a concentration of 5, 10, or 20 μg/ml. The concentration of quercetin (Sigma Co.), which was a positive control known as an anti-elastase substance, was 10 μg/ml or 20 μg/ml in the test. The reaction was carried out in 96-well UV plates (Corning Co.) at room temperature for 20 minutes. The absorbance was measured at 405 nm at intervals of 1 minute using a microplate spectrophotometer (Bio-Rad). The enzyme activity was determined by obtaining the maximal slope of absorbance over time. All enzymatic reactions were repeatedly carried out three times, and expressed as average values. The anti-elastase activity of Irilin B is shown in Table 2. The anti-collagenase activity was calculated as below:

[Calculation Formula]

Anti-elastase activity (%) = [(absolute value of slope in control - absolute value of slope in test sample) / absolute value of slope in control] X 100

[Table 2]

| Sample name | Concentration (μg/ml) | Enzyme activity (Absolute value of maximal slope of absorbance over time at 405 nm) | Anti-elastase activity (%) |
|---|---|---|---|
| Irilin B | 5 | 0.85 | 32.54±2.04 |
| | 10 | 0.72 | 42.86±2.89 |
| | 20 | 0.54 | 57.14±4.22 |
| Positive control (Quercetin) | 10 | 0.88 | 30.16±3.14 |
| | 20 | 0.77 | 38.89±2.67 |
| Control (DMSO) | 20 | 1.26 | - |

As can be seen from the results of Table 1 above, Irilin B showed anti-elastase activity (32.54%, 42.86%, and 57.14%) in a dose dependent manner (5, 10, and 20 μg/ml). It was confirmed that Irilin B showed significantly excellent anti-elastase activity (by about 1.47-fold) compared with quercetin (38.89%, 20 μg/ml), a positive control, tested at the same concentration in the same conditions. That is, it could be seen that Irilin B showed excellent anti-elastase activity, and thus can be used for skin anti-aging cosmetics for skin moisturization and wrinkle reduction.

**Example 4: Investigation of expression levels of skin moisturizing factors (AQP3 and HAS2)**

In order to investigate the expression levels of aquaporin 3 (AQP3) and hyaluronic acid synthase 2 (HAS2), which are moisturizing factors involved in the migration of cell moisture, the test was performed using the human skin keratinocytes HaCaT cells (Korean Cell Line Bank). The HaCaT cells were incubated in Dulbecco's modified Eagle's medium (DEME, Gibco 1210-0038) containing 10% FBS. Cell incubation was carried out using a 5% $CO_2$ incubator at 37°C. After the cells were treated with Irilin B and ultraviolet light (aging treatment: UVA: 5 J/cm$^2$), the levels of AQP3 and HAS2 expressed in the cells were measured. The expression levels were measured by extracting RNA from the HaCaT cells and performing real-time polymerase change reaction (RT-PCR). The test substance Irilin B was treated after UVA irradiation. Irilin B was in advance prepared into a 10 mg/ml stock solution by addition of DMEM. Upon the test, the cells were treated with the stock solution diluted to 1/1,000 to 1/500. That is, the final treatment concentration of Irilin B was 10-20 μg/ml, and the cell treatment time was 24 hours.

The gene expression test (RNA extraction and real-time polymerase chain reaction (PCR)) was performed in the following order:

i) RNA was extracted using TRIzol Reagent (Gibco, USA).
ii) The cDNA synthesis from the extracted RNA was performed using the ReverTra Ace reverse transcriptase kit (Toyobo, USA).
iii) For comparative measurement of the expressions of the HaCaT cell markers, RT-PCR (Applied Biosystems, USA) was performed.

The particular Taqman Gene expression assay IDs used in the test were hyaluronic acid synthase 2 (HAS2): Hs00193435_m1; aquaporin 3 (AQP3): Hs01105469_g1; and glyceraldehyde phosphodihydrogenase (GAPDH): Gap014231257_s1, respectively. The expressions of genes were identified through RT-PCR using the synthesized cDNA, and the primers used in the test are shown in Table 3.

[Table 3]

| Gene | SEQ ID NO | Name | Sequence (5'-> 3') |
|---|---|---|---|
| AQP3 | 1 | AQP3_forward | AGACAGCCCCTTCAGGATTT |
| | 2 | AQP3_reverse | TCCCTTGCCCTGAATACTGG |
| HAS2 | 3 | HAS2_forward | TAAGGTGTTGTGTGTGACTG |
| | 4 | HAS2_reverse | CAGAATCCAAACAGACAGTTC |
| GAPDH | 5 | GAPDH_forward | ACCACAGTCCATGCCATCAC |
| | 6 | GAPDH_reverse | TCCACCACCCTGTTTCTTTA |

The HaCaT cells were treated with Irilin B at different concentrations (10, 20, 40 μg/ml)after UVA irradiation, and then RT-PCR was performed using the primers in Table 3. The reaction conditions were denaturation at 95°C for 30 min, and 45 cycles of 5 seconds at 95°C and 20 seconds at 60°C. Thereafter, annealing to 95°C at 0.2°C/15 sec was carried out, and then the reaction was stopped.

As can be seen in FIG. 1, the expression of the moisturizing factor AQP3 was reduced due to photo-aging in the HaCaT cells irradiated with UVA. Irilin B restored the UVA-reduced level of AQP3 in a dose dependent manner. Especially, the expression level of APQ3 in the HaCaT cells treated with Irilin B at 40 μg/ml was again increased to a level in the control irradiated without UVA.

In FIG. 2, the expression of the moisturizing factor HAS2 was reduced when the HaCaT cells were irradiated with UVA, and was again restored to a level in the normal control by treatment with Irilin B in a dose dependent manner. These results suggest that Irilin B can be used for a skin anti-aging cosmetic product restoring the reduction of moisturization due to skin aging.

**Example 5: Keratinocyte differentiation promoting effect**

Human skin keratinocytes (Korean Cell Line Bank) were seeded in the $CO_2$ incubation flask in which DMEM containing 10% FBS was placed. After the cells were grown to a confluency of about 80%, Irilin B at different concentrations (5, 10, 20, 50, 100 μg/ml)were added to the cells, followed by incubation for 3 days. Thereafter, the cells were treated with a reduction modifier, such as sodium dodecyl sulfate (SDS) and β-mercaptoethanol, to remove proteins. After the

treatment, the remaining keratinocyte layer was suspended in distilled water, and then the absorbance was measured at 310 nm to analyze the peptide concentration. The increase by each sample was compared on the basis of the negative control, distilled water (0%). In the positive control, the cells were treated with 10 nM TGF-β known as a cell differentiation factor. All the tests of keratinocyte differentiation promoting effect were repeatedly carried out three times. The absorbance at 310 nm is expressed as a mean and standard deviation, and the cell differentiation promoting effect is expressed, using the following calculation formula, as an average value (%), in Table 4.

[Calculation Formula]

Cell differentiation promoting effect (%) = (absorbance value at 310 nm in test sample - absorbance value at 310 nm in negative control) X 100

[Table 4]

| Sample name | Concentration (μg/ml) | Absorbance (310 nm) | Cell differentiation promoting effect (%) |
|---|---|---|---|
| Irilin B | 10 | 0.839±0.05 | 28.2 |
| | 20 | 1.024±0.06 | 46.7 |
| | 50 | 1.510±0.04 | 95.3 |
| | 100 | 1.981±0.08 | 142.4 |
| Positive control (TGF-β) | 10 nM | 2.004±0.03 | 144.7 |
| Negative control (distilled water) | - | 0.557±0.01 | 0 |

As can be seen from the results in Table 4, it was confirmed that Irilin B promoted the differentiation of keratinocytes in the tested concentration sections (5, 10, 20, 50, and 100 μg/ml)in a dose dependent manner. Especially, the treatment with 100 μg/ml Irilin B showed an excellent cell differentiation promoting effect equivalent to the cell differentiation factor TGF-β (10 nM) used as the positive control. Therefore, it was confirmed that Irilin B retains skin moisture, thereby maintaining moisturization and suppressing skin wrinkle formation, and thus can be used as a raw material of a cosmetic product for skin aging prevention.

## Claims

1. A skin anti-aging composition containing Irilin B (5,7,2'-trihydroxy-6-methoxyisoflavone) represented by chemical formula 1 below or a salt thereof.

[Chemical Formula 1]

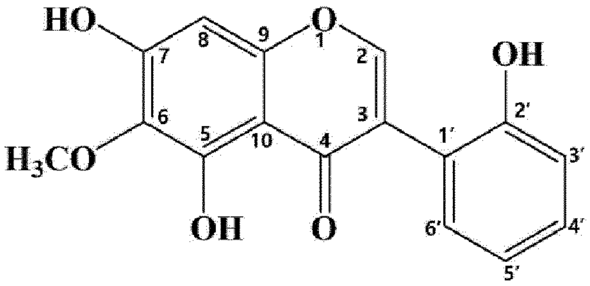

2. The skin anti-aging composition of claim 1, wherein the composition is used for skin wrinkle reduction, skin wrinkle prevention, skin elasticity enhancement, or skin moisturization.

3. The skin anti-aging composition of claim 1, wherein the composition is a cosmetic composition.

4. The skin anti-aging composition of claim 1, wherein the composition is a food or feed composition.

5. The skin anti-aging composition of claim 1, wherein the composition is a pharmaceutical composition.

Fig 1

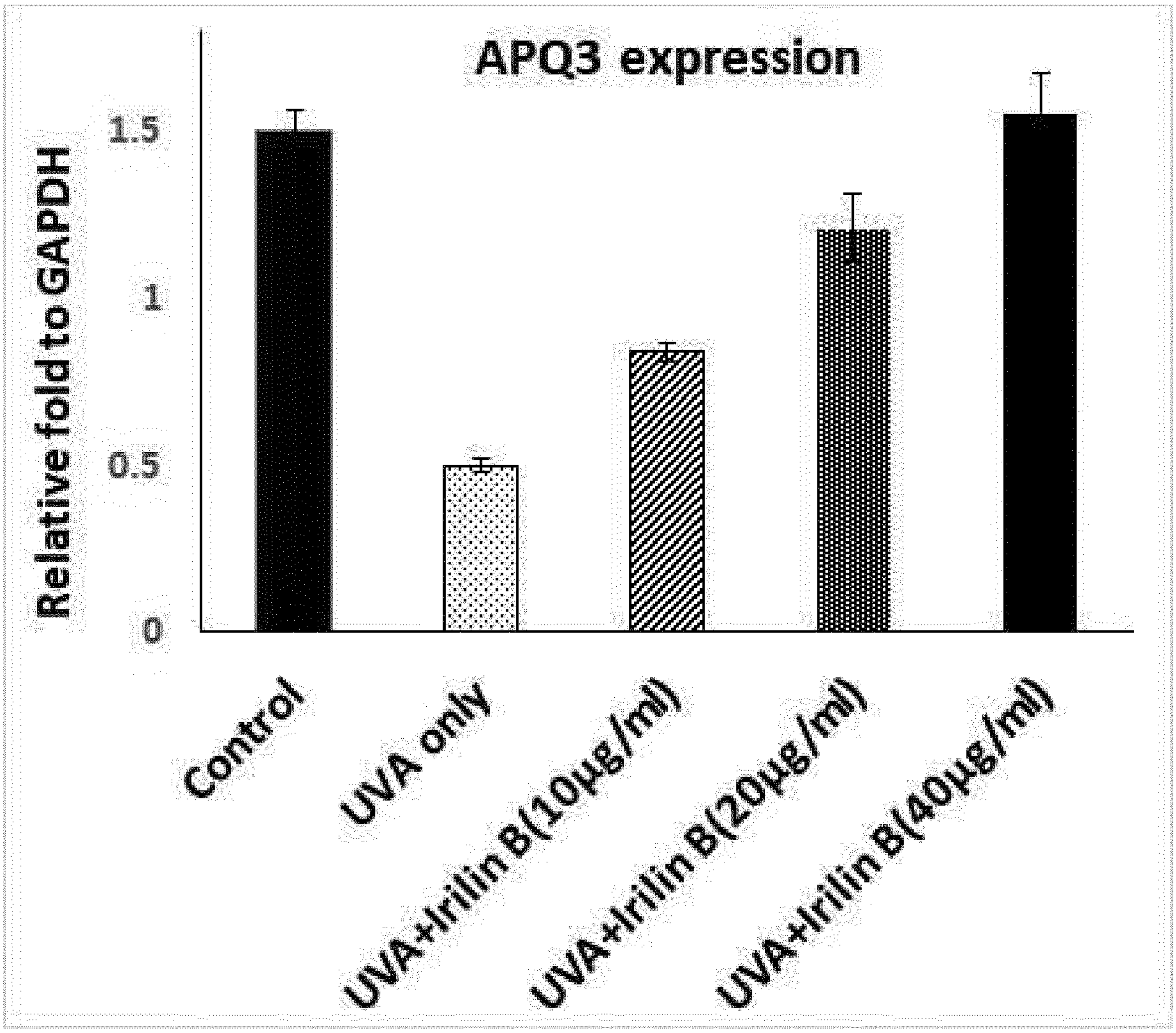
APQ3 expression
Relative fold to GAPDH
0
0.5
1
1.5
Control
UVA only
UVA+Irilin B(10μg/ml)
UVA+Irilin B(20μg/ml)
UVA+Irilin B(40μg/ml)

Fig 2

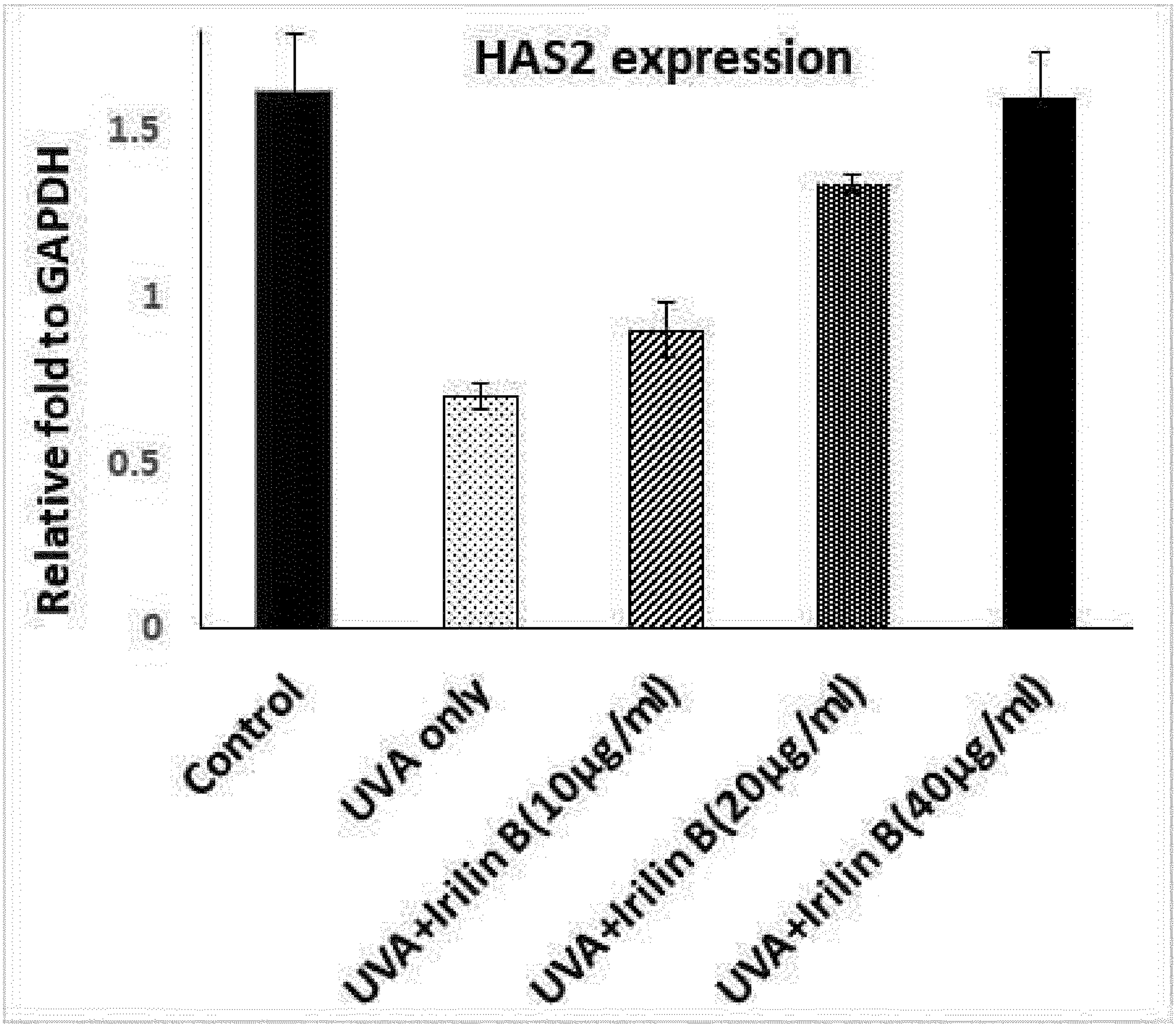
HAS2 expression
Relative fold to GAPDH
1.5
1
0.5
0
Control
UVA only
UVA+Irilin B(10µg/ml)
UVA+Irilin B(20µg/ml)
UVA+Irilin B(40µg/ml)

## INTERNATIONAL SEARCH REPORT

International application No.
PCT/KR2019/005652

A. CLASSIFICATION OF SUBJECT MATTER

*A61K 8/49(2006.01)i, A23L 33/10(2016.01)i, A23K 20/121(2016.01)i, A61K 31/352(2006.01)i, A61Q 19/08(2006.01)i, A61P 17/00(2006.01)i*

According to International Patent Classification (IPC) or to both national classification and IPC

B. FIELDS SEARCHED

Minimum documentation searched (classification system followed by classification symbols)
A61K 8/49; A23K 20/116; A61K 8/97; A61Q 19/00; A61Q 19/08; A23L 33/10; A23K 20/121; A61K 31/352; A61P 17/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: irilin, Salicornia, Salicornia europaea, antiaging, cosmetic

C. DOCUMENTS CONSIDERED TO BE RELEVANT

| Category* | Citation of document, with indication, where appropriate, of the relevant passages | Relevant to claim No. |
|---|---|---|
| Y | KR 10-1812319 B1 (PHYTO CORPORATION) 28 December 2017<br>See abstract; paragraphs [0018]-[0020]; and claims 1, 4, 8. | 1-5 |
| Y | KR 10-1363413 B1 (DANJOUNGBIO CO., LTD.) 14 February 2014<br>See abstract; paragraph [0023]; and claim 1. | 1-5 |
| Y | KR 10-2008-0096132 A (SAENG GREEN CO., LTD.) 30 October 2008<br>See abstract; and claims 1, 2. | 1-5 |
| Y | KR 10-2012-0086438 A (COSMECCA KOREA CO., LTD.) 03 August 2012<br>See abstract; and claims 1, 10. | 1-5 |
| Y | KR 10-2011-0013812 A (THEFACESHOP CO., LTD.) 10 February 2011<br>See abstract; and claim 1. | 1-5 |

☐ Further documents are listed in the continuation of Box C.    ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
02 SEPTEMBER 2019 (02.09.2019)

Date of mailing of the international search report
**02 SEPTEMBER 2019 (02.09.2019)**

Name and mailing address of the ISA/KR
Korean Intellectual Property Office
Government Complex Daejeon Building 4, 189, Cheongsa-ro, Seo-gu, Daejeon, 35208, Republic of Korea
Facsimile No. +82-42-481-8578

Authorized officer

Telephone No.

Form PCT/ISA/210 (second sheet) (January 2015)

**INTERNATIONAL SEARCH REPORT**
Information on patent family members

International application No.
**PCT/KR2019/005652**

| Patent document cited in search report | Publication date | Patent family member | Publication date |
|---|---|---|---|
| KR 10-1812319 B1 | 28/12/2017 | None | |
| KR 10-1363413 B1 | 14/02/2014 | CN 104274355 A<br>CN 104274355 B | 14/01/2015<br>12/04/2017 |
| KR 10-2008-0096132 A | 30/10/2008 | None | |
| KR 10-2012-0086438 A | 03/08/2012 | None | |
| KR 10-2011-0013812 A | 10/02/2011 | KR 10-1124971 B1 | 27/03/2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 8231370 A
- JP 8208424 A
- JP 9040523 A
- JP 10036283 A
- KR 101812319

### Non-patent literature cited in the description

- Remington's Pharmaceutical Sciences. 1995